# EUROPÄISCHE PATENTSCHRIFT

(11) **EP 2 524 705 B1**
(45) Veröffentlichungstag und Bekanntmachung des Hinweises auf die Patenterteilung: **23.04.2014**
(21) Anmeldenummer: 11004166.2
(22) Anmeldetag: 19.05.2011
(51) Int. Cl.: A61L 15/24, A61L 15/44, A61L 15/46, A61L 15/60, A61F 13/00, A61K 8/43, C07C 279/26

(54) **Sterile Wundauflage mit einem synthethischen Dreiblock-Elastomer und einem hydrophorbierten polymeren Biguanid**
Sterilized wound dressing comprising a triblock-elastomeric component and hydrophobic polymeric Biguanid
Pansement stérile comprenant un composé élastomère tribloc et un polymère hydrophobe de Biguanid

(43) Veröffentlichungstag der Anmeldung: 21.11.2012
(73) Patentinhaber: Lohmann & Rauscher GmbH & Co. KG, 56567 Neuwied (DE)
(72) Erfinder: Gorka, Marius-Thomas, 56218 Mülheim-Kärlich (DE)
(74) Vertreter: Leinweber & Zimmermann

(56) Entgegenhaltungen:
- WO-A1-2007/000590
- FR-A1- 2 916 356
- US-A1- 2009 155 339

## Beschreibung

Die Erfindung betrifft eine Wundauflage mit einem Träger und einer antimikrobiell wirksamen Wundkontaktschicht sowie ein Material zum Herstellen einer Wundkontaktschicht für eine Wundauflage.

Antiseptische Wundauflagen werden für die lokale Behandlung von traumatischen Wunden, wie etwa Schnitt- und Schürfwunden, und chronischen Wunden, wie etwa Dekubitus, Ulcus cruris u. dgl., Verbrennungen und postoperativen Wunden eingesetzt. Eine antimikrobielle Ausstattung verleiht der Wundauflage eine lokale, antimikrobielle Wirksamkeit. Die so ausgestattete Wundauflage kann auf infektionsgefährdeten, kritisch kolonisierten und infizierten Wunden bei Menschen und Tieren angewendet werden.

Angesichts der wachsenden Anforderungen an hygienische Standards im medizinischen und klinischen Bereich besteht ein steigender Bedarf an antiseptischen Wundauflagen, Verbandstoffen und Materialien. Dabei müssen gleichzeitig hohe Anforderungen an die antimikrobielle Wirksamkeit und die Bioverträglichkeit derartiger Materialien erfüllt werden.

Bekannte Antibiotika bzw. Breitbandantibiotika sowie antimikrobiell wirksame Stoffe, wie Triclosan oder Silber in jeglicher Form, sind hinsichtlich der antimikrobiellen Wirksamkeit problematisch, weil Resistenzen der pathogenen Keime gegen diese Wirkstoffe ausgebildet werden, so daß die antimikrobielle Wirksamkeit nicht mehr im gewünschten Umfang besteht.

Seit den 1990er Jahren wird Polyhexanid (Polyhexamethylenbiguanid; PHMB), ein weit verbreitetes Desinfektionsmittel mit antibakterieller, antimykotischer, antiviraler und antiprotozoischer Wirksamkeit, auch bei therapeutischen Anwendungen, z. B. in der Chirurgie, Augenheilkunde und zur Wundbehandlung, vermehrt eingesetzt. PHMB ist ein Polykation, dessen antibakterielle Wirksamkeit auf der Interaktion mit negativ geladenen Phospholipiden von Bakterienzellwänden beruht. PHMB weist als Antiseptikum ein sehr breites Wirkungsspektrum auf und ist z. B. auch gegen Hefen und Methicillin-resistente Staphylococcus aureus (MRSA) Stämme wirksam. Gleichzeitig ist die Gewebeverträglichkeit von PHMB als sehr gut zu bewerten, da die neutralen Lipide von humanen Zellen mit PHMB kaum eine Wechselwirkung eingehen. Auch im Vergleich zu anderen Antiseptika ist PHMB sehr gut bioverträglich (vgl. Polyhexanide: A safe and highly effective biocide, K. Kaehn, Tabelle 3, Toxikologische Daten bekannter Antiseptika im Vergleich zu PHMB). Darüber hinaus ist festzustellen, daß zu PHMB derzeit keine Resistenzbildungen bekannt sind.

PHMB findet Anwendung als Breitbandspektrum-Bakterizid in Körperpflegeanwendungen (DE 698 17 654 T2), industriellen Anwendungen sowie in Medizinprodukten und ist als wäßrige Lösung in Form eines Hydrochloridsalzes kommerziell erhältlich (Cosmocil CQ, Cosmocil PQ, Fa. Arch). Für die meisten Anwendungen ist die Verwendung von PHMB als hydrophiles, gut in Wasser lösliches Hydrochloridsalz gut geeignet. Ähnliches Verhalten zeigen andere Antiseptika, wie Chlorhexidin (1,1'-Hexamethylenbis[5-(4-chlorphenyl)-biguanid]-digluconat) und Alexidin (1,1'-Hexamethylenbis[5-(4-(2-ethylhexyl)phenyl)-biguanid]-diacetat).

In der EP 127 229 B1 ist eine antiseptische Kompresse beschrieben, die eine Wundkontaktschicht aus einer elastomeren Matrix, einem unpolaren Öl, einem Hydrokolloid (Natriumcarboxymethylcellulose) als Dispersion und mind. einem Tensid (Tween 80), ggf. mit mind. einem antimikrobiellen Mittel (Silbersulfadiazin), aufweist. Bei Einsatz entsprechender Kompressen werden Resistenzbildungen beobachtet. Ferner ist die Verwendung von Tensiden problematisch, weil sie ein gewisses zytotoxisches Potential besitzen. Als problematisch kann es weiterhin angesehen werden, daß Silbersulfadiazin verschreibungspflichtig ist.

In der EP 1 691 851 B1 ist ein Wundverband beschrieben, der ein mit Öffnungen versehenes flüssigkeitsdurchlässiges Substrat und eine absorbierende (mindestens 50 % der Trockenmasse) nichthaftende Polymerzusammensetzung aufweist, die eine hydrophobe organische Polymermatrix, einen Weichmacher und hydrophile organische Mikroteilchen sowie ggf. bioaktive Mittel enthält. In der genannten Schrift werden synthetische und natürliche bioaktive Mittel beschrieben. Beim Einsatz dieser Mittel hat es sich als problematisch erwiesen, daß das Absorptionsmittel (Superabsorber) im Verband die Flüssigkeit in Form eines Gels bindet. Durch den Quellungsvorgang werden die Öffnungen für Exsudat (z. B. in dickflüssiger Form) nahezu unpassierbar. Ein freier Abtransport des Exsudats in den saugenden Sekundärverband kann dann behindert werden.

In der WO 0203899 ist ein mit einer wäßrigen, hydrophilen PHMB-Variante imprägnierter Verband beschrieben. Beim Einsatz dieses Verbands wird beobachtet, daß der Träger mit der Wunde verklebt, wobei es auch zum Einwachsen von Granulationsgewebe kommen kann. Daher ist das in der genannten Schrift beschriebene Produkt trotz der guten Verträglichkeit von PHMB und der fehlenden Resistenzbildung für die Wundbehandlung nur bedingt geeignet.

Das Verkleben des Verbands mit der Wunde kann durch Einsatz einer sterilen, nichtklebenden Kompresse gemäß der EP 1 143 895 B1 verhindert werden. Diese Kompresse enthält eine elastomere Matrix aus einem Dreiblockelastomer, einem öligen Weichmacher, Vaseline und hydrophilen Teilchen eines Hydrokolloids (CMC, Alginat) und ggf. einen Wirkstoff. Als Wirkstoffe werden in der genannten Schrift Silbersulfadiazin, Antibiotika, wie bspw. Neomyzin oder Polymyzin) und nichtsteroide oder steroide Entzündungshemmer, wie bspw. Triamcinolonacetonide, vorgeschlagen. Diese Wirkstoffe weisen einen nur begrenzten Nutzen auf.

Die WO 2007/000590 A1 offenbart einen PHMB-Silber-Komplex, mit dem Silber in gewünschten Oxidationsstufen bereitgestellt werden soll. Gemäß der genannten Schrift kann dieser Komplex als Beschichtung für medizinische Elemente eingesetzt werden.

Die FR 2 916 356 A1 offenbart ein Mittel, welches die Freisetzung eines Wirkstoffs aus einer einen öligen Körper aufweisenden Wundauflage ermöglicht.

Die US 2009/0155339 A1 offenbart ein biokompatibles Fasermaterial mit einer antimikrobiellen Ausstattung, welches als bioresorbierbares Nahtmaterial zu medizinischen Zwecken eingesetzt werden kann. Gemäß dieser Schrift kann die Wirkungsdauer und die Wirkung der mikrobiellen Ausstattung durch Einsatz verschiedener PHMB-Typen eingestellt werden, wobei ein PHMB-Typ hydrophobiert sein, insbes. als Stearat vorliegen kann.

Angesichts der vorstehend beschriebenen Probleme im Stand der Technik liegt der Erfindung die Aufgabe zugrunde, eine bioverträgliche, nichtklebende (ggf. leicht haftende) Wundauflage mit einer unspezifischen, aber dennoch hoch wirksamen antimikrobiellen Ausrüstung für die Behandlung von infektionsgefährdeten, kritisch kolonisierten und infizierten Wunden zur Verfügung zu stellen.

Erfindungsgemäß wird diese Aufgabe durch eine Weiterbildung der bekannten Wundauflagen gelöst, die im Wesentlichen dadurch gekennzeichnet ist, daß die Wundkontaktschicht einen hydrophobierten Wirkstoff aufweist, mit dem eine elastomere Matrix der Wundkontaktschicht ausgestaltet ist.

Durch Hydrophobieren des Wirkstoffs wird es möglich, die Wundkontaktschicht als organische polymere Matrix auszuführen. Dadurch kann das Verkleben des Trägers mit der Wunde bzw. das Einwachsen von Granulationsgewebe vermieden werden. Gleichzeitig kann der hydrophobierte Wirkstoff homogen in der Wundkontaktschicht verteilt werden. Dementsprechend kann die Wundkontaktschicht einer erfindungsgemäßen Wundauflage eine mit dem hydrophobierten Wirkstoff ausgestattete elastomere Matrix aufweisen.

Bei einer besonders bevorzugten Ausführungsform der Erfindung weist der hydrophobierte Wirkstoff hydrophobiertes oligomeres/polymeres Biguanid, insbesondere hydrophobiertes PHMB und/oder hydrophobiertes oligomeres/polymeres Guanid auf. Dabei wird von der Erkenntnis Gebrauch gemacht, daß kommerziell erhältliche wasserlösliche Salze von PHMB zum Erhalt einer homogenen Lösung/Mischung/Dispersion in einer hydrophoben organischen elastomeren Polymermatrix nicht geeignet sind. Das gilt im besonderen dann, wenn die Verarbeitungstemperaturen über dem Siedepunkt des Wassers bzw. der wäßrigen PHMB-Lösung liegen. Wegen der Hydrophilie des PHMB-Hydrochloridsalzes ist der Wirkstoff auch in wasserfreier Form (Pulver, Teilchen, Partikel, Mikropartikel) ungeeignet, um eine homogene und phasenstabile Mischung in der hydrophoben elastomeren Polymermatrix sicherzustellen. Der Schmelzbereich von PHMB*HCl Partikeln (Pulver) liegt bei ca. 79 bis 136 °C, was in etwa der Verarbeitungstemperatur entspricht. Bei diesen Temperaturen (Verarbeitungstemperaturen von 110 bis 150 °C) kommt es zu einer Phasentrennung, begleitet von Agglomeratbildung und heterogener Verteilung des Wirkstoffs in der polymeren Matrix.

Durch die Hydrophobierung des Wirkstoffs läßt sich eine homogene Verteilung des Antiseptikums in der hydrophoben Masse ohne Zusatz von Tensiden erreichen. Dabei kann die Hydrophobierung durch die Verminderung der positiven Ladung des polykationischen Wirkstoffs erfolgen (Deprotonierung), oder durch Austausch des hydrophilen Gegenions/Anions (Chlorid) gegen ein hydrophoberes Gegenion/Anion.

Im Rahmen der Erfindung wurde herausgefunden, daß PHMB in Form eines Salzes einer organischen Säure, die 5 bis 25 Kohlenstoffatome enthält, aufgrund der Hydrophobie und eines gesenkten Schmelzbereichs (z. B. PHMB-Stearat: 71,2 bis 86,7 °C) sehr gut geeignet ist, um homogen in polymere Elastomermassen eingearbeitet zu werden. Dabei bleibt die hohe antimikrobielle Wirksamkeit erhalten.

Die homogene Verteilung des Wirkstoffs in der elastomeren Matrix, und damit in dem beschichteten Produkt, gewährleistet eine flächig homogene Freisetzung des Wirkstoffs in die Wunde und vermeidet das lokale Aufkonzentrieren des Wirkstoffs und damit verbundene lokal auftretende zytotoxische Effekte. Andererseits werden zu geringe Wirkstoffkonzentrationen in gewissen Regionen der Wundauflage vermieden, die zu einer Wirkungslücke sowie ggf. zu einer bakteriellen Besiedlung der Wundauflage und damit Gefährdung des Patienten führen könnten.

Die Wundkontaktschicht einer erfindungsgemäßen Wundauflage umfaßt vorzugsweise eine organische polymere Matrix, einen Weichmacher (*plasticizer*) und hydrophile organische bzw. anorganische Mikropartikel, die bei Kontakt mit wäßriger Lösung ein Gel bilden.

Die polymere Matrix kann ein Dreiblockcopolymer A-B-A enthalten. Dabei enthält die polymere Matrix vorzugsweise nicht mehr als 3,2 Gewichtsteile, insbes nicht mehr als 2,6 Gewichtsteile eines Blockpolymers, wobei der terminale Block A vom Polystyroltyp und der zentrale Block B vom gesättigten Polyolefintyp sein kann und der Styrenanteil zwischen 25 und 40 % beträgt. Zweckmäßig werden hydrierte Copolymere Polystyren-Polyethylen-Polybutylen-Polystyrene (SEBS, z. B. G1651, Kraton) eingesetzt. Im Rahmen der Erfindung werden hydrierte Copolymere Polystyren-b-Poly(ethylen/propylen)-b-Polystyrene (SEEPS, Septon 4055, Kuraray) bevorzugt. Zusätzlich oder alternativ kann die Wundkontaktschicht einer erfindungsgemäßen Wundauflage eine mit dem hydrophobierten Wirkstoff versetzte einphasige Salbe, wie etwa Vaseline, ein hydrophobes Basisgel DAB und /oder mehrphasiges (z.B. Wasser-in-Öl) System und/oder ein mit dem hydrophobierten Wirkstoff versetztes hydrophobes Silikongel, aufweisen.

Zur Förderung der Heilung ohne Risiko von mikrobiellen Verschmutzungen werden Wundauflagen üblicherweise sterilisiert. Wundinfektionen verzögern nachweislich die Heilung. Sie werden durch pathogene Keime verursacht, die in die Wunde (ggf. auch über die Wundauflage) eindringen, sich dort vermehren und dabei Giftstoffe erzeugen, die sowohl auf das Wundgewebe als auch auf den gesamten Organismus wirken.

Es sind eine Vielzahl von Techniken zum Zerstören von verunreinigenden Mikroorganismen bekannt. Neben der Sterilisation durch gesättigten Dampf oder durch trockene Wärme wird routinemäßig die Sterilisation durch Gas (Ethylenoxid, Formaldehyd) oder die Sterilisation durch Bestrahlung angewendet. Jedoch ist keine dieser Techniken für die Herstellung von Produkten vorbehaltlos geeignet, welche eine fetthaltige elastomere Matrix enthalten. Das betrifft im besonderen Produkte zur pharmazeutischen Anwendung. Für diese Produkte ist die Sterilisation durch gesättigten Dampf oder Trockenwärme kaum anwendbar, weil die elastomere Matrix und das Hydrokolloid die hohen Temperaturen und/oder eine erhöhte Luftfeuchtigkeit nur schlecht vertragen. Die Sterilisation durch Gas wird wegen des dabei auftretenden Risikos, daß Rückstände in der Wundauflage verbleiben, als problematisch angesehen. Außerdem ist es bei dieser Technik im allgemeinen nicht möglich, eine Verteilung des Sterilisationsmittels über das gesamte Volumen der elastomeren Polymermasse zu erhalten. Dadurch wird die Wirksamkeit dieser Sterilisation begrenzt.

Im allgemeinen verhindert auch der Einsatz einer Sterilbarriere (primärer Packstoff), der die Sterilität des Produkts bei der Vermarktung bis zu der Anwendung am Patienten bewahrt, die Anwendung der meisten o. g. Sterilisationsverfahren, da der Packstoff als gasdicht ausgewählt wurde, um den oxidierenden Einfluß des Luftsauerstoffs sowie den Einfluß der Luftfeuchtigkeit auf das in der Matrix dispergierte hygroskopische Hydrokolloid zu unterbinden.

Aus den genannten Gründen kommt üblicherweise eine Sterilisation durch Bestrahlung zum Einsatz. Dadurch wird eine wirksame Sterilisation bis ins Innere des Produkts sichergestellt. Es können zwei Strahlungsarten verwendet werden, nämlich β- und γ -Strahlung.

Die Sterilisationsdosis wird als Funktion der anfänglichen mikrobiologischen Belastung (*Bioburden*), d. h. der Menge an Keimen, die vor der Sterilisation auf/im Produkt vorhanden sind, im Rahmen einer Dosisbestimmung angepaßt.

Zum Erhalt einer wirksamen Dekontamination mit einer ausreichenden Sicherheitsspanne wird auf die zu sterilisierenden Produkte im allgemeinen eine mittlere Dosis von 25 kGray angewendet. In der Praxis erhält ein Produkt eine Menge, die zwischen 25 und 40 kGray entsprechend dem verwendeten Verfahren variiert.

Es hat sich allerdings gezeigt, daß die Sterilisation durch Bestrahlung einen Einfluß auf die so behandelte elastomere Matrix hat. Insbesondere ist die Energie, die durch die Strahlung in die Matrix eingebracht wird, ausreichend hoch, um die Verbindungen Kohlenstoff-Kohlenstoff und Kohlenstoff-Wasserstoff der verwendeten Elastomere zu brechen und ggf. Unterbrechungen der Ketten in diesen polymeren Makromolekülen und Verminderungen ihrer mittleren molekularen Masse hervorzurufen, die Ihre Eigenschaften, insbesondere ihre Kohäsionsfähigkeit, beeinflussen bzw. herabsetzen. Folglich sind diese Produkte entweder aufgrund von Schwierigkeiten in Verbindung mit der Handhabung beim Anlegen oder Abnehmen des Verbands oder aufgrund eines relativ hohen Polymer-Preises oder einer gewissen Gewebeunverträglichkeit der elastomeren Polymere oder auch durch den Verlust der Kohäsion nach der Strahlensterilisation nicht vollkommen zufriedenstellend.

Im Rahmen der Erfindung hat es sich als besonders vorteilhaft erwiesen, wenn die Wundkontaktschicht zur Lösung dieser Probleme eine elastomere Matrix beinhaltet, die weniger als 3,2 Gew.-% eines Elastomers, insbes. 3,0 Gew.-% oder weniger, vorzugsweise weniger als 2,7 Gew.-% eines Polymeranteils enthält, wodurch neben dem wirtschaftlichen Vorteil eine bessere Gewebeverträglichkeit erhalten wird.

Zum Erhalt einer besonders großen Sterilisationsbeständigkeit werden bei einer besonders bevorzugten Ausführungsform der Erfindung Elastomere bzw. Elastomermischungen mit besonders hohem Molekulargewicht eingesetzt, um den negativen Auswirkungen der Strahlensterilisation auf die Kohäsion der Matrix entgegenzuwirken. Als hochmolekulare Polymere können erfindungsgemäß unter dem Handelsnamen Septon 4055 vertriebene Elastomere mit einem Molekulargewicht von 200.000 bis 300.000 Dalton eingesetzt werden. Überraschenderweise wurde festgestellt, daß die Zugabe von ultrahochmolekularen Elastomeren, wie unter dem Handelsnamen Septon 4077 vertriebene Polymere, mit einem Molekulargewicht von 400.000 bis 450.000 Dalton die Kohäsion nach der Sterilisation erheblich verbessert. Bedingt durch den Inhalt an relativ temperaturempfindlichen Komponenten, wie Vaseline oder CMC, in der Matrix, ist die Höhe der Verarbeitungstemperatur begrenzt. Sie liegt vorzugsweise nicht höher als etwa 140 bis 145 °C. Die mit dem Einsatz eines ultrahochmolekularen Elastomers (Mw = ca. 400.000 bis 450.000 Dalton) erwartete Steigerung der Verarbeitungstemperatur ist aber nur geringfügig und liegt im akzeptablen Bereich.

Im Sinne der Sicherstellung einer guten Verarbeitbarkeit hat es sich im Rahmen der Erfindung als besonders zweckmäßig erwiesen, wenn der Gesamtpolymeranteil der elastomeren Matrix mindestens 50 % eines hochmolekularen Elastomers mit einem Molekulargewicht von etwa 200.000 bis 300.000 Dalton enthält, wobei der Rest aus einem ultrahochmolekularen Polymer mit einem Molekulargewicht von 400.000 bis 450.000 Dalton bestehen kann.

Zur Vermeidung von Problemen bei der Verarbeitung entsprechender Elastomere hat es sich als vorteilhaft erwiesen, wenn das Molekulargewicht der ultrahochmolekularen Polymere weniger als 600.000 Dalton, insbes. weniger als 550.000 Dalton, beträgt.

Die Brookfield-Viskosität erfindungsgemäß eingesetzter Polymere beträgt zweckmäßigerweise mindestens 5.000 mPas (für eine 10%ige Lösung in Toluol bei 30 °C).

Unter den Produkten/Weichmachern, die zur Plastifizierung des Elastomers gut geeignet sind, kann insbes. auf bei Raumtemperatur flüssige oder feste Fettkörper, insbes. Paraffinöle, medizinische Weißöle, Mineralöle, Salbenparaffine, Vaseline, Silikonöle oder Silikonfette bzw. Wachse hingewiesen werden, sowie auf Mischungen davon. Bevorzugt werden Weichmacher, wie Vaseline, deren Tropfpunkt zwischen 35 und 70 °C liegt. Bevorzugt werden auch medizinische Weißöle, deren Reinheitsanforderungen der Ph. Eur. entsprechen.

Als hydrophile organische bzw. anorganische Mikropartikel, die Wasser binden und dabei gelieren, können z. B. die allgemein bekannten Hydrokolloide (CMC, Alginate, Gelatine, Xanthane, Pektine), aber auch Silikate, wie Bentonite, Aerosile oder Superabsorber, eingesetzt werden. Bevorzugt werden Mikropartikel mit einem Durchmesser von 50 bis 300 µm (unter Annahme einer sphärischen Form), insbes. mit einem Durchmesser von 50 bis 200 µm.

Die Matrix kann auch Antioxidantien enthalten. Als geeignete Antioxidantien können die Schwefel-Antioxidantien genannt werden, wie zum Beispiel das von der Firma Akzo Nobel chemicals unter der Verkaufsbezeichnung PERKACIT ZDBC vermarktete Zink-Dibutyldithiocarbamat, und/oder die Phenol-Antioxidantien, wie zum Beispiel die unter den Verkaufsbezeichnungen IRGANOX®1010, IRGANOX®565, IRGANOX®1035 von der Firma BASF vermarkteten Produkte.

Im Rahmen der vorliegenden Erfindung wird die Verbindung aus IRGANOX®1010 bevorzugt.

Die Wundkontaktschicht einer erfindungsgemäßen Wundauflage kann ferner ein Additiv umfassen, das aus der Gruppe bestehend aus einem weiteren Stabilisator, Extrudierhilfe, Füllstoff, Pigment, Farbstoff, Vernetzungsmittel, geruchshemmenden Mittel, Klebrigmacher, Verträglichkeitsvermittler und Kombination davon ausgewählt ist.

Die organische Säure, die das Salz des polymeren Biguanids bildet, kann eine Phosphon-, Phosphor-, Sulfon- oder Schwefelsäuregruppe enthalten, enthält jedoch vorzugsweise eine Carbonsäuregruppe. Die organische Säure kann aromatisch sein, ist jedoch vorzugsweise aliphatisch, einschließlich alicyclisch. Wenn die organische Säure aliphatisch ist, kann die aliphatische Kette der organischen Säure linear oder verzweigt, gesättigt oder ungesättigt sein, einschließlich Gemische davon. Vorzugsweise ist die aliphatische Kette linear und es ist auch bevorzugt, daß die organische Säure eine aliphatische Carbonsäure ist. Mit aliphatischen Carbonsäuren wird die beste Hydrophobierung erreicht.

Es ist bevorzugt, daß die organische Säure nicht weniger als 8, bevorzugter nicht weniger als 10, insbes. nicht weniger als 12 Kohlenstoffatome, ausschließlich der Säuregruppe, enthält. Vorzugsweise enthält die organische Säure nicht mehr als 24, bevorzugter nicht mehr als 20 und insbes. nicht mehr als 18 Kohlenstoffatome, ausschließlich der Säuregruppe. Die organische Säure kann mehr als eine Säuregruppe enthalten, jedoch ist es bevorzugt, daß nur eine solche Gruppe vorliegt. Sofern die organische Säure mehr als eine Säuregruppe enthält, wird der Effekt der Hydrophobierung entsprechend abgeschwächt. Die organische Säure kann mit einem Halogen oder insbes. einer Hydroxygruppe substituiert sein. Im Sinne einer möglichst wirkungsvollen Hydrophobierung hat es sich jedoch als günstig erwiesen, wenn die organische Säure frei von Substituenten ist.

Einige aliphatische Carbonsäuren sind als Gemische, wie jene, erhalten von tierischen Fetten und Pflanzenölen, kommerziell erhältlich und diese enthalten sowohl gesättigte als auch ungesättigte aliphatische Ketten. Diese können sich auch als nützlich erweisen, insbes. die C₁₄-₁₈-Alkylcarbonsäuren und deren vollständig gesättigte oder hydrierte Analoge. Beispiele für ggf. substituierte Carbonsäuren sind Valerian-, Hexan-, Octan-, 2-Octen-, Laurin-, 5-Dodecen-, Myristin-, Pentadecan-, Palmitin-, Öl-, Stearin-, Eicosan-, Heptadecan-, Palmitolein-, Rizinolein-, 12-Hydroxystearin-, 16-Hydroxyhexadecan-, 4-Hydroxydecan-, Dodecandi-, Undecandi-, Sebacin-, Benzoe-, Hydroxybenzoe- und Terephthalsäuren.

Als besonders günstig hat es sich erwiesen, wenn die aliphatische Carbonsäure Stearinsäure und das polymere Biguanid PHMB darstellt. Die Umsetzung von PHMB mit Stearinsäure zu PHMB-Stearat kann ohne nennenswerte Beeinflussung der antimikrobiellen Eigenschaften des PHMB erfolgen.

Das polymere Biguanid enthält mindestens drei Biguanideinheiten. Vorzugsweise enthält das polymere Biguanid mehr als zwei Biguanideinheiten, die durch eine Brückengruppe gebunden sind, die mindestens eine Methylengruppe enthält. Vorzugsweise ist die Brückengruppe derart ausgelegt, daß es mindestens drei und nicht mehr als zehn und insbes. nicht mehr als acht Kohlenstoffatome, die sich zwischen den zwei benachbarten Biguanideinheiten befinden, gibt. Das polymere Biguanid kann durch eine geeignete Gruppe beendet sein, die eine Kohlenwasserstoff- oder substituierte Kohlenwasserstoffgruppe oder ein Amin sein kann. Das erfindungsgemäß zum Einsatz kommende polymere Biguanid enthält mindestens 3 Biguanideinheiten, vorzugsweise zwischen 7 und 18, besonders bevorzugt nicht weniger als 9 und nicht mehr als 17. Es handelt sich vorzugsweise um ein lineares polymeres Biguanid.

Im Falle des bevorzugten Poly(hexamethylenbiguanids) ist es ein durch die Verbindungen der Formel 1 wiedergegebenes Gemisch in der freien Basenform, worin der Wert von n 4 bis 40 und insbes. 4 bis 15 beträgt. Es ist besonders bevorzugt, daß der Mittelwert von n in dem Gemisch 12 ist. Vorzugsweise entspricht das mittlere Molekulargewicht des Polymergemisches n = 10 - 13.

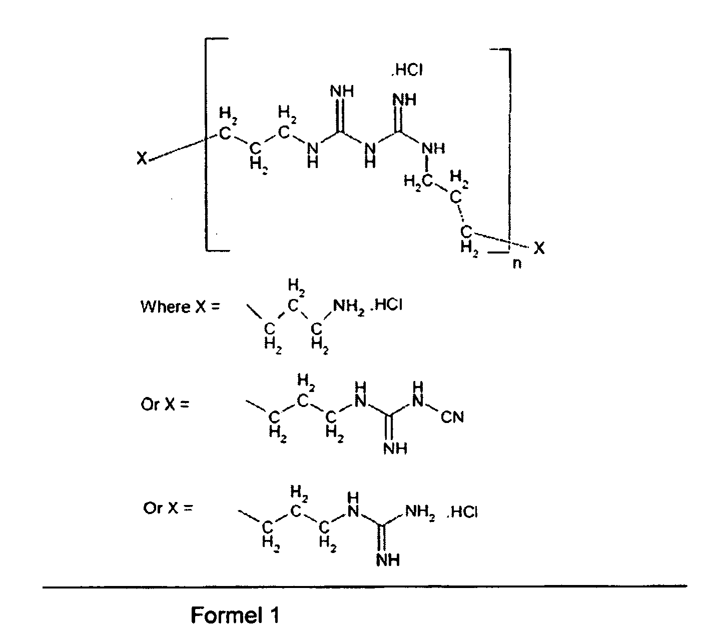

Gemäß der vorliegenden Erfindung wird eine Zusammensetzung bereitgestellt, die eine Trägermasse und ein polymeres Biguanid in Form seines Salzes mit einer organischen Säure, die 4 bis 30 Kohlenstoffatome enthält, einschließlich Gemische davon zur Verwendung in medizinischen Formulierungen, umfaßt.

Gemäß einer bevorzugten Ausführungsform erfindungsgemäßer Wundauflagen ist die antimikrobielle Ausstattung zumindest auf einem Teil der Oberfläche in Form einer Beschichtung oder als eine integrierte Komponente der Beschichtungsmasse ausgeführt. Vorzugsweise erstreckt sich die antimikrobielle Ausrüstung über die gesamte Oberfläche des Produkts.

Bei einer besonders bevorzugten Ausführungsform der Erfindung besitzt die elastomere Matrix eine antimikrobielle Imprägnierung.

Bei Einsatz erfindungsgemäßer Wundauflagen ist PHMB frei verfügbar und diffusionsfähig in das beschädigte Körpergewebe.

Wie der vorstehenden Erläuterung erfindungsgemäßer Wundauflagen zu entnehmen ist, umfaßt ein Material zum Herstellen der Wundkontaktschicht dieser erfindungsgemäßen Wundauflage im wesentlichen einen hydrophobierten Wirkstoff, welcher in einer elastomeren Matrix, einer einphasigen Salbe, wie etwa Vaseline, in einem hydrophoben Basisgel DAB und/oder in einem mehrphasigen (z.B. Wasser-in-Öl) System und/oder in einem hydrophoben Silikongel, verteilt ist.

Nachstehend wird die Herstellung erfindungsgemäßer Wundauflagen erläutert:

### PHMB-Stearat kann wie folgt erhalten werden:

Überführung des hydrophilen PHMB Hydrochloridsalzes im Rahmen einer Metathesereaktion in ein hydrophobes PHMB-Stearatsalz, dabei entsteht nur ein harmloses NaCl-Salz.

| | **Komponente** | **Produktname** | **Menge** | |
|---|---|---|---|---|
| 1 | Stearinsäure | - | 30,0 g | (105,5 mmol) |
| 2 | Natriumhydroxid | - | 3,7 g | (92,4 mmol) |
| 3 | Polyhexamethylenbiguanid (Polyhexanid) | Cosmocil PQ (20 %, Fa. Arch) | 100,0 g | (ca. 7,7 mmol) |
| 4 | Wasser AP nach Ph. Eur. | - | 300 ml | |

### Durchführung:

Stearinsäure (Fa. Merck) in 300 ml AP Wasser vorlegen, Natriumhydroxid zugeben und auf ca. 80 °C erhitzen. Cosmocil PQ (Fa. Arch) mit Wasser verdünnen (1:1), langsam zugeben und 2 Std. bei ca. 80 °C rühren. Niederschlag abfiltrieren und mit Wasser waschen. Niederschlag über Nacht bei ca. 40 °C trocknen bzw. lyophilisieren.

### PHMB-Laurat kann wie folgt erhalten werden:

Die Überführung des PHMB-Hydrochloridsalzes in ein PHMB-Stearatsalz erfolgt wie oben beschrieben. Allerdings wird anstelle von 30,0 g Stearinsäure 21,0 g Laurinsäure (Fa. Merck) eingesetzt.

### Erfindungsgemäße Wundauflagen können wie folgt erhalten werden:

### Beispiel 1:

| | Menge [g] | Gewichtsteile [%] |
|---|---|---|
| Paraffinöl | 1120 | 71,6 |
| PHMB-Stearat | 56 | 3,58 |
| Copolymer | 41 | 2,62 |
| Antioxidans | 1,6 | 0,10 |
| Vaseline | 125 | 7,99 |
| CMC | 221 | 14,13 |
| Summe | 1564,6 | 100,0 |

Die Masse wird in einem Labordissolver hergestellt. 1120 g des Paraffinöls werden vorgelegt und mit 41 g eines Elastomer-Copolymers SEEPS (Septon 4055, Fa. Kuraray) und 1,6 g eines Antioxidans (Irganox 1010) versetzt und bei ca. 135 °C bis zur Erhaltung einer homogenen klaren elastomeren Masse gerührt. Nach der Einarbeitung von 125 g Vaseline (Vara AB, Fa. Sasol) werden 56 g des PHMB-Stearats in Pulverform zugegeben und homogen eingerührt. Im Anschluß werden 221 g der Natrium-Carboxymethylcellulose (CMC, Blanose 7H4XF, Fa. Aqualon) zugesetzt. Die so erhaltene elastomere Masse wird noch etwa 30 min. gerührt, bis eine homogene Masse erhalten wird.

Die Masse kann ebenfalls in einem Kneter oder ähnlichen für Verarbeitung von Hotmelt-Massen allg. bekannten Anlagen/Geräten hergestellt werden.

Die Masse kann in einem Tauchbad-Verfahren bei ca. 140-145°C auf das Gewebe (Gittertüll) aufgebracht werden, so daß das textile Gebilde vollständig umhüllt ist, die Zwischenräume/Poren aber weitgehend offen bleiben, um den Durchfluß des Exsudats zu gewährleisten.

### Beispiel 2:

Analog Bsp. 1, allerdings wurde ein Polymergemisch aus 2 % Septon 4055 und 0,6 % Septon 4077 eingesetzt.

### Beispiel 3:

Analog Bsp. 1, allerdings wurden 15 % eines nichtionischen Cellulose-Derivats (HPMC, Bonucel D15000) eingesetzt.

### Beispiel 4:

Analog Bsp. 1, allerdings wurden anstelle von PHMB-Stearat 3,58 % eines deprotonierten (durch Zusatz von einer Base, z. B. NaOH) PHMB eingesetzt.

### Beispiel 5:

Analog Bsp. 1 bis 4, allerdings wurde statt PHMB-Stearat das PHMB-Laurat eingesetzt.

Die Erfindung ist nicht auf die vorstehend erläuterten Ausführungsbeispiele beschränkt. Vielmehr ist auch an den Einsatz von Trägermaterialien in Form von offenmaschigen Gewirken und Geweben und/oder Polyurethan-Schäumen wie etwa Vivo MCF 03 (Fa. AMS) gedacht. Die Konzentration des antimikrobiell wirksamen Mittels kann in Abhängigkeit vom Einsatzbereich verändert werden. Entscheidend ist, daß das antimikrobiell wirksame Mittel hydrophobiert ist. Dabei kann die erfindungsgemäße Wundauflage auch mindestens einen weiteren, vorzugsweise ebenfalls hydrophobierten Wirkstoff wie etwa Octenidin-Steartat, und/oder Metalle wie Silber, Kupfer, Selen und/oder Metallverbindungen, insbesondere Metallsalze (Ag₂O, AgCl, ZnO, MgO), aufweisen.

Im Rahmen der Erfindung kann es von besonderer Bedeutung sein, daß die Wundauflagen nur im Bereich von 5% bis 30% der Trockenmasse absorbieren (gemeint ist mit dem beschränkten Absorptionsbereich die elastomere Wundkontaktschicht (die Trägermaterialien können für die gesamte Wundauflage zu anderen Ergebnissen führen)). Dadurch wird die Gelbildung und Quellung (durch hydrokolloide Lösungen) eingeschränkt, so daß der Exsudattransport durch die Öffnungen des Verbands in den Sekundärverband nicht behindert wird.

Im Rahmen der Erfindung kann dem Gesichtspunkt besondere Bedeutung zukommen, daß der Träger offenmaschig ist, so daß nach der Beschichtung/Umhüllung der den Träger bildenden Fasern mit der elastomeren Masse noch ein Exsudatdurchfluß gewährleistet ist.

## Patentansprüche

1. Wundauflage mit einem Träger und einer antimikrobiell wirksamen und eine elastomere Matrix aufweisenden Wundkontaktschicht, **dadurch gekennzeichnet, daß** die Wundkontaktschicht einen hydrophobierten Wirkstoff aufweist, mit dem die elastomere Matrix ausgestaltet ist.

2. Wundauflage nach Anspruch 1, **dadurch gekennzeichnet, daß** der hydrophobierte Wirkstoff hydrophobiertes polymeres Biguanid, insbesondere hydrophobiertes PHMB und/oder Guanide, aufweist.

3. Wundauflage nach Anspruch 2, **dadurch gekennzeichnet, daß** das hydrophobierte PHMB ein Salz einer vorzugsweise 5 bis 25 Kohlenstoffatome enthaltenden organischen Säure und/oder ein Gemisch davon aufweist.

4. Wundauflage nach Anspruch 3, **dadurch gekennzeichnet, daß** der hydrophobierte Wirkstoff einen Schmelzbereich unterhalb von 120 °C, vorzugsweise zwischen 71,2 und 86,7 °C, aufweist.

5. Wundauflage nach Anspruch 3 oder 4, **dadurch gekennzeichnet, daß** das hydrophobierte PHMB PHMB-Stearat aufweist.

6. Wundauflage nach einem der vorhergehenden Ansprüche, **dadurch gekennzeichnet, daß** die Wundkontaktschicht mindestens einen weiteren, vorzugsweise hydrophobierten Wirkstoff und/oder Metalle und/oder Metallverbindungen, insbesondere Metallsalze, aufweist.

7. Wundauflage nach einem der vorhergehenden Ansprüche, **dadurch gekennzeichnet, daß** der Träger ein vorzugsweise offenmaschiges Gewirk, Gewebe und/oder Vlies und/oder einen Polyurethan-Schaum aufweist.

8. Wundauflage nach einem der vorhergehenden Ansprüche, **dadurch gekennzeichnet, daß** die elastomere. Matrix durch ein synthetisches Dreiblock-Elastomer, vorzugsweise ein Copolymer aus Polystyrolblock und Polyolefinblock (SEPS, SEBS, SEEPS), gebildet wird, oder durch Mischungen davon, wobei der Gesamtpolymeranteil unter 3,2 Gew.-%, vorzugsweise 2,6 Gew.% oder weniger, beträgt und durch ein unpolares Öl plastifiziert ist.

9. Wundauflage nach Anspruch 8, **dadurch gekennzeichnet, daß** die elastomere Matrix ein synthetisches Dreiblock-Elastomer mit hohem Molekulargewicht und einer Brookfield-Viskosität von 5.000 mPas oder mehr (für eine 10%ige Lösung bei 30 °C) oder Mischungen entsprechender Polymere enthält.

10. Wundauflage nach Anspruch 9, **dadurch gekennzeichnet, daß** das Molekulargewicht des Dreiblock-Elastomers 150.000 Dalton oder mehr, insbes. 200.000 Dalton oder mehr, vorzugsweise 300.000 Dalton oder mehr, besonders bevorzugt 400.000 bis 450.000 Dalton oder mehr, aber weniger als 600.000 Dalton beträgt.

11. Wundauflage nach einem der vorhergehenden Ansprüche, **dadurch gekennzeichnet, daß** die elastomere Matrix ein ionisches oder nichtionisches Hydrokolloid beinhaltet, das homogen in der Matrix dispergiert ist.

12. Wundauflage nach einem der vorhergehenden Ansprüche, **dadurch gekennzeichnet, daß** der Wirkstoff homogen in der elastomeren Matrix gelöst/dispergiert/verteilt ist.

13. Wundauflage nach einem der vorhergehenden Ansprüche, **dadurch gekennzeichnet, daß** die elastomere Matrix bereichsweise keinen Wirkstoff enthält.

14. Wundauflage nach einem der vorhergehenden Ansprüche, **dadurch gekennzeichnet, daß** die elastomere Matrix einen Tackifier enthält, um die Klebkraft zu erhöhen.

15. Wundauflage nach einem der vorhergehenden Ansprüche, **dadurch gekennzeichnet, daß** die Wundkontaktschicht eine mit dem hydrophobierten Wirkstoff ausgestattete einphasige Salbe, wie etwa Vaseline, ein hydrophobes Basisgel DAB und /oder mehrphasiges (z.B. Wasser-in-Öl) System und/oder ein mit dem hydrophobierten Wirkstoff versetztes hydrophobes Silikongel, aufweist.

16. Material zum Herstellen einer Wundkontaktschicht einer Wundauflage nach einem der vorhergehenden Ansprüche.

## Claims

1. A wound dressing comprising a backing and an antimicrobially effective wound contact layer having an elastomer matrix, **characterised in that** the wound contact layer has a hydrophobised active ingredient with which the elastomer matrix is formed.

2. The wound dressing according to Claim 1, **characterised in that** the hydrophobised active ingredient has a hydrophobised polymeric biguanide, in particular hydrophobised PHMB and/or guanides.

3. The wound dressing according to Claim 2, **characterised in that** the hydrophobised PHMB has a salt of an organic acid containing preferably 5 to 25 carbon atoms and/or a mixture thereof.

4. The wound dressing according to Claim 3, **characterised in that** the hydrophobised active ingredient has a melting range below 120°C, preferably between 71.2 and 86.7°C.

5. The wound dressing according to Claim 3 or 4, **characterised in that** the hydrophobised PHMB has PHMB stearate.

6. The wound dressing according to any of the preceding claims, **characterised in that** the wound contact layer has at least one further, preferably hydrophobised active ingredient and/or metals and/or metal compounds, in particular metal salts.

7. The wound dressing according to any of the preceding claims, **characterised in that** the backing has a preferably open mesh knitted fabric, a woven and/or non-woven fabric and/or a polyurethane foam.

8. The wound dressing according to any of the preceding claims, **characterised in that** the elastomer matrix is formed by a synthetic three-block elastomer, preferably a copolymer composed of a polystyrene block and a polyolefin block (SEPS, SEBS, SEEPS) or by mixtures thereof, the total polymer content being less than 3.2 % by weight, preferably 2.6 % by weight or less, and being plasticised by a non-polar oil.

9. The wound dressing according to Claim 8, **characterised in that** the elastomer matrix contains a synthetic three-block elastomer with a high molecular weight and a Brookfield viscosity of 5,000 mPas or more (for a 10 % solution at 30°C) or mixtures of corresponding polymers.

10. The wound dressing according to Claim 9, **characterised in that** the molecular weight of the three-block elastomer is 150,000 daltons or more, in particular 200,000 daltons or more, preferably 300,000 daltons or more, particularly preferably 400,000 to 450,000 daltons or more, but less than 600,000 daltons.

11. The wound dressing according to any of the preceding claims, **characterised in that** the elastomer matrix contains an ionic or non-ionic hydrocolloid that is dispersed homogeneously in the matrix.

12. The wound dressing according to any of the preceding claims, **characterised in that** the active ingredient is dissolved/dispersed/distributed homogeneously in the elastomer matrix.

13. The wound dressing according to any of the preceding claims, **characterised in that** the elastomer matrix does not contain any active ingredient in some areas.

14. The wound dressing according to any of the preceding claims, **characterised in that** the elastomer matrix contains a tackifier in order to increase the adhesive strength.

15. The wound dressing according to any of the preceding claims, **characterised in that** the wound contact layer has a single phase ointment provided with the hydrophobised active ingredient, such as for example petroleum jelly, a hydrophobic base gel DAB and/or a multi-phase (e.g. water in oil) system and/or a hydrophobic silicon gel displaced with the hydrophobised active ingredient.

16. A material for producing a wound contact layer of a wound dressing according to any of the preceding claims.

## Revendications

1. Pansement comportant un support, ainsi qu'une surface de contact avec la plaie à action antimicrobienne et présentant une matrice élastomère, **caractérisé en ce que** la surface de contact avec la plaie présente une substance active hydrofugée dont est pourvue la matrice élastomère.

2. Pansement selon la revendication 1, **caractérisé en ce que** la substance active hydrofugée présente du biguanide polymère hydrofugé, notamment du guanide et/ou du PHMB hydrofugé.

3. Pansement selon la revendication 2, **caractérisé en ce que** le PHMB hydrofugé présente un sel d'un acide organique contenant de préférence 5 à 25 atomes de carbone et/ou un mélange de tels sels.

4. Pansement selon la revendication 3, **caractérisé en ce que** la substance active hydrofugée présente un intervalle de fusion inférieur à 120 °C, de préférence situé entre 71,2 et 86,7 °C.

5. Pansement selon la revendication 3 ou 4, **caractérisé en ce que** le PHMB hydrofugé présente du stéarate de PHMB.

6. Pansement selon l'une des revendications précédentes, **caractérisé en ce que** la surface de contact avec la plaie présente au moins une autre substance active, de préférence hydrofugée, et/ou des métaux et/ou composés métalliques, notamment des sels métalliques.

7. Pansement selon l'une des revendications précédentes, **caractérisé en ce que** le support présente un tricot, un tissu et/ou un non-tissé à mailles de préférence ouvertes et/ou une mousse polyuréthane.

8. Pansement selon l'une des revendications précédentes, **caractérisé en ce que** la matrice élastomère est constituée par un élastomère tribloc synthétique, de préférence un copolymère à base d'un bloc polystyrène et d'un bloc polyoléfinique (SEPS, SEBS, SEEPS), ou par des mélanges de ceux-ci, la part de polymères totale étant située sous 3,2 % en poids, en étant de préférence de 2,6 % en poids ou moins, et est plastifiée par une huile apolaire.

9. Pansement selon la revendication 8, **caractérisé en ce que** la matrice élastomère contient un élastomère tribloc synthétique présentant un poids moléculaire élevé et une viscosité Brookfield de 5 000 mPa.s ou plus (pour une solution à 10 % à 30 °C) ou des mélanges de polymères correspondants.

10. Pansement selon la revendication 9, **caractérisé en ce que** le poids moléculaire de l'élastomère tribloc est de 150 000 daltons ou plus, notamment de 200 000 daltons ou plus, de préférence de 300 000 daltons ou plus, en allant de manière particulièrement préférentielle de 400 000 à 450 000 daltons ou plus, en restant toutefois inférieur à 600 000 daltons.

11. Pansement selon l'une des revendications précédentes, **caractérisé en ce que** la matrice élastomère contient un hydrocolloïde ionique ou non ionique en dispersion homogène dans la matrice.

12. Pansement selon l'une des revendications précédentes, **caractérisé en ce que** la substance active est dissoute/dispersée/répartie de manière homogène dans la matrice élastomère.

13. Pansement selon l'une des revendications précédentes, **caractérisé en ce que** la matrice élastomère ne contient aucune substance active par endroits.

14. Pansement selon l'une des revendications précédentes, **caractérisé en ce que** la matrice élastomère contient un agent poisseux visant à accroître le pouvoir collant.

15. Pansement selon l'une des revendications précédentes, **caractérisé en ce que** la surface de contact avec la plaie présente un onguent monophasique pourvu de la substance active hydrofugée, tel que par exemple de la vaseline, un gel de base hydrofugé DAB et/ou un système polyphasique (p. ex. eau dans huile) et/ou un gel de silicium hydrofugé additionné de la substance active hydrofugée.

16. Matériau de production d'une surface de contact avec la plaie d'un pansement selon l'une des revendications précédentes.
